# EUROPÄISCHE PATENTSCHRIFT

(11) **EP 3 642 848 B1**
(45) Veröffentlichungstag und Bekanntmachung des Hinweises auf die Patenterteilung: **29.10.2025**
(21) Anmeldenummer: 18734781.0
(22) Anmeldetag: 20.06.2018
(51) Int. Cl.: G16H 30/20, G16H 30/40

(54) **VERFAHREN ZUR ERZEUGUNG EINES ZWEIDIMENSIONALEN GESAMTBILDS**
METHOD FOR PRODUCING A TWO-DIMENSIONAL WHOLE IMAGE
PROCÉDÉ POUR GÉNÉRER UNE IMAGE GLOBALE BIDIMENSIONNELLE

(30) Priorität: 20.06.2017 DE 102017210222
(43) Veröffentlichungstag der Anmeldung: 29.04.2020
(73) Patentinhaber: Sirona Dental Systems GmbH, 64625 Bensheim (DE)
(72) Erfinder: ADAMSON, Anders, 64297 Darmstadt (DE); ERBACHER, Markus, 68229 Mannheim (DE); HAUTH, Steffen, 55246 MZ-Kostheim (DE); VOSS, Björn, 69117 Heidelberg (DE)
(74) Vertreter: Aldridge, Henry Alexander
(86) Internationale Anmeldenummer: PCT/EP2018/066459
(87) Internationale Veröffentlichungsnummer: WO 2018/234396

(56) Entgegenhaltungen:
- DE-A1- 102014 207 667

## Beschreibung

### Technisches Gebiet

Die Erfindung betrifft ein Verfahren zur Erzeugung eines zweidimensionalen Gesamtbilds eines mittels mehrerer Einzelbilder erfassten Aufnahmebereichs, wobei jedes Einzelbild einen Teilbereich des Aufnahmebereichs aus einer eigenen Blickrichtung und mit einem eigenen Abstand zu dem Teilbereich erfasst und zumindest eine Vielzahl der Einzelbilder entsprechend einer ermittelten jeweiligen räumlichen Ausrichtung zueinander zu dem Gesamtbild zusammengesetzt werden.

### Stand der Technik

Aus dem Stand der Technik sind eine Vielzahl von Verfahren bekannt, die es ermöglichen ein Objekt bzw. einen Gesamtaufnahmebereich mittels einer Kamera zu erfassen, auch wenn das Objekt bzw. der Gesamtaufnahmebereich deutlich größer als das Messfeld der Kamera ist. Die Verfahren basieren darauf, mehrere Einzelbilder zu einem Gesamtbild zusammenzufügen. DE102014207667A1 offenbart ein Verfahren zur Durchführung einer optischen dreidimensionalen Aufnahme nach dem Präambel des Hauptanspruchs der vorliegenden Erfindung.

Beispiele hierfür sind übliche Panoramafunktionen von digitalen Kameras. Die mehreren erzeugten Einzelbilder werden im Panorama-Modus typischerweise auf eine Kugeloberfläche projiziert, um sie zu einem Gesamtbild zusammenzufügen. Dies ist möglich, da davon ausgegangen werden darf, dass die Kamera während der Einzelaufnahmen im Wesentlichen nur gedreht wird, sich die dreidimensionale Kameraposition jedoch im Verhältnis zur Objektgröße bzw. zur Größe des Aufnahmebereichs nur unwesentlich ändert.

Aufgabe der vorliegenden Erfindung ist es, den Stand der Technik weiter zu entwickeln. Insbesondere soll ein alternatives Verfahren zum Erzeugen eines Gesamtbilds aus Einzelbildern bereitgestellt werden, welches trotz einer translatorischen Bewegung der Kamera relativ zum Objekt/Aufnahmebereich zuverlässig Gesamtbilder mit einer hohen Aufnahmequalität liefert.

Darüber hinaus sollen Verzerrungen im zweidimensionalen Gesamtbild verringert werden.

### Darstellung der Erfindung

Ein Gegenstand der Erfindung ist ein Verfahren zur Erzeugung eines zweidimensionalen Gesamtbilds eines mittels mehrerer Einzelbilder erfassten Aufnahmebereichs, wobei jedes Einzelbild einen Teilbereich des Aufnahmebereichs aus einer eigenen Blickrichtung und mit einem eigenen Abstand zu dem Teilbereich erfasst, jedes Einzelbild eine Vielzahl von Bildpunkten mit mindestens einem Farbwert und/oder mindestens einem Grauwert und/oder mindestens einem Höhenwert umfasst und der in jedem Einzelbild jeweils erfasste Teilbereich mit mindestens einem Teilbereich in jeweils mindestens einem weiteren Einzelbild überlappt. Eine räumliche Ausrichtung einer Hauptbildebene jedes Einzelbilds zu einer Hauptbildebene des jeweiligen weiteren Einzelbilds wird anhand der Überlappung der jeweils erfassten Teilbereiche ermittelt und zumindest eine Vielzahl der Einzelbilder werden entsprechend der ermittelten jeweiligen räumlichen Ausrichtung zueinander zu dem Gesamtbild zusammengesetzt.

Die räumliche Ausrichtung der Hauptbildebene jedes Einzelbilds zu der Hauptbildebene des weiteren Einzelbilds wird durch Optimieren eines Qualitätswerts ermittelt, wobei für eine erste relative räumliche Ausrichtung zu mindestens einem ersten Bildpunkt in der Hauptbildebene des Einzelbilds ein gemäß der ersten Ausrichtung entsprechender Bildpunkt in der Hauptbildebene des weiteren Einzelbilds ermittelt wird, ein Vergleichswert der jeweiligen Farbwerte und/oder Grauwerte des Bildpunkts des Einzelbilds und des entsprechenden Bildpunkts des weiteren Bilds gebildet wird, aus dem mindestens einen Vergleichswert der Qualitätswert gebildet wird und der Qualitätswert durch Verändern der relativen räumlichen Ausrichtung der Hauptbildebene des Einzelbilds zu der Hauptbildebene des weiteren Einzelbilds einem vorgegebenen Zielwert zumindest angenähert wird.

DieEinzelbilderwerdenmittels einer Aufnahmeeinheit aufgenommen, wobei die Aufnahmeeinheit relativ zu dem aufzunehmenden Objekt oder Bereich bewegbar ist. Entsprechend der Position der Aufnahmeeinheit zu dem aufzunehmenden Bereich oder Objekt während des Erzeugens eines Einzelbilds erfasst das Einzelbild einen Teilbereich aus einer bestimmten Raumrichtung, hier als Blickrichtung bezeichnet, und für einen bestimmten Abstand zwischen Aufnahmeeinheit und Teilbereich.

Es versteht sich, dass alle Einzelbilder zu dem Gesamtbild zusammengefügt werden oder das Gesamtbild alternativ aus einem ausgewählten Teil der erzeugten Einzelbilder gebildet wird. Als Hauptbildebene wird jeweils eine dem jeweiligen zweidimensionalen Einzelbild zugeordnete Ebene im dreidimensionalen Raum bezeichnet, z.B. eine innerhalb des Einzelbilds ermittelte schärfste Ebene oder eine nominelle Fokusebene entsprechend der Nennbrennweite einer zur Aufnahme des Einzelbilds verwendeten Kamera.

Die relative räumliche Ausrichtung gibt die Position bzw. die Orientierung der Hauptbildebene eines ersten Einzelbilds im dreidimensionalen Raum relativ zu einem zweiten Einzelbild an. Aufgrund der Ausrichtung kann eine Transformation in ein gemeinsames Koordinatensystem bzw. eine Projektion des einen Einzelbilds auf das andere Einzelbild bzw. in die Hauptbildebene des anderen Einzelbilds vorgenommen werden.

Ausgangspunkt des Optimierungsverfahrens zur Auffindung der relativenAusrichtung zwischen zwei Einzelbildern ist jeweils eine erste relative räumliche Ausrichtung zwischen dem ersten und dem weiteren Einzelbild. Als erste Ausrichtung wird beispielsweise eine Identität der Hauptbildebenen der zwei zueinander auszurichtenden Einzelbilder angenommen.

Es versteht sich, dass die Veränderung der relativen räumlichen Ausrichtung während des Optimierungsverfahrens keinen Einschränkungen unterliegt, also sechs Freiheitsgrade aufweist.

Ferner versteht es sich, dass vorteilhafterweise für jede relative räumliche Ausrichtung alle Punkte in der Hauptbildebene des Einzelbilds ermittelt werden, zu denen ein gemäß der relativen räumlichen Ausrichtung entsprechender Bildpunkt in der Hauptbildebene des weiteren Einzelbilds existiert. Ferner wird vorteilhafterweise zu jedem Paar aus Bildpunkt des Einzelbilds und entsprechendem Bildpunkt des weiteren Bilds ein Vergleichswert berechnet und alle berechneten Vergleichswerte dem Qualitätswert zugrunde gelegt.

Entspricht die im Rahmen des Optimierungsverfahrens angenommene relative räumliche Ausrichtung der realen räumlichen Ausrichtung der Hauptbildebenen zueinander, so geben der Bildpunkt und der sich entsprechende Bildpunkt jeweils denselben Bereich des aufzunehmenden Objekts/Bereichs wieder bzw. haben diesen erfasst und die Werte des Bildpunkts und des entsprechenden Bildpunkts stimmen überein oder weichen nur geringfügig voneinander ab.

Der Fortgang des Optimierungsverfahrens wird durch Vergleich mit dem Zielwert überprüft. Der Zielwert ist beispielsweise ein zu erreichender Grenzwert oder weicht von einem zu erreichenden Grenzwert um einen vorgegebenen Wert ab.

Ein Vorteil des erfindungsgemäßen Verfahrens ist, dass die Ausrichtung zwischen den Einzelbildern ohne weitere Hilfsmittel wie Marker oder in den Bildern identifizierte Strukturen und ohne weitere Annahmen/Näherungen, wie beispielsweise eine translatorisch unveränderte Kameraposition, bestimmt wird. Das Optimierungsverfahren bzw. der Qualitätswert basieren allein auf den Bilddaten selbst, also den Werten der Bildpunkte der Einzelbilder. Es wird ausgenutzt, dass sich die Pixelwerte von Bildpunkten, welche denselben Objektbereich erfassen, ähneln oder sogar gleichen und zwar unabhängig davon, ob die Pixelwerte Farbwerte oder Grauwerte repräsentieren.

Das mindestens eine Bildpunktpaar wird durch eine Projektion des Einzelbilds und des weiteren Einzelbilds oder eines das weitere Einzelbild umfassenden Zwischenbilds auf eine Gesamtbildfläche ermittelt, wobei die Projektion entsprechend der relativen räumlichen Ausrichtung sowie einer relativen räumlichen Ausrichtung des Einzelbilds oder des weiteren Einzelbilds zu der Gesamtbildfläche vorgenommen wird. Die Gesamtbildfläche ist die Oberfläche eines Torus.

Mittels des erfindungsgemäßen Verfahrens ist ein zweidimensionales Gesamtbild mit besonders hoher Auflösung erzeugbar. Insbesondere ist die Auflösung des erfindungsgemäßen 2D-Gesamtbilds deutlich besser als die Auflösung eines zweidimensionalen Gesamtbilds, welches durch eine Projektion eines beispielsweise mittels intraoraler Kamera erzeugten dreidimensionalen Gesamtbilds erzeugt wird.

Weiterhin sind Verzerrungen im zweidimensionalen Gesamtbild verringert.

Vorteilhafterweise sind die Einzelbilder jeweils ein Farbbild einer Kamera oder Grauwertbildeiner Kamera oder dreidimensionales Bild mit einer Textur. Als Textur wird typischerweise ein Abbild der Oberflächenbeschaffenheit bezeichnet.

Dabei kann mindestens ein Bildpunktepaar aus Bildpunkt des Einzelbilds und entsprechendem Bildpunkt des weiteren Einzelbilds durch eine Projektion des Einzelbilds in die Hauptbildebene des weiteren Einzelbilds oder durch eine Projektion des weiteren Einzelbilds indie Hauptbildebene des Einzelbilds ermittelt werden, wobei die Projektion entsprechend der relativen räumlichen Ausrichtung vorgenommen wird.

Alternativ wird das Bildpunktepaar durch eine Projektion des Einzelbilds auf eine Bildebene eines das weitere Einzelbild umfassenden Zwischenbilds oder durch eine Projektion eines das weitere Bild umfassenden Zwischenbilds auf die Hauptbildebene des Einzelbilds ermittelt.

Durch eine der relativen räumlichen Ausrichtung entsprechenden Projektionbzw. Transformation werden die Bilder in eine gemeinsame Bildebenebzw. auf eine gemeinsameBildflächetransformiert. Durch die Projektion bzw. Transformation fallen Bildpunkte des Einzelbilds und des weiteren Einzelbilds zusammen, wobei zusammenfallende bzw. aufeinander abgebildete Bildpunkte jeweils ein Bildpunktepaar bilden.

Als Projektion wird hinsichtlich aller vorgenannten Alternativen eine projektive Transformation bezeichnet.

Das Gesamtbild wird schrittweise durch Hinzufügen des Einzelbilds zueinem zumindest das weitereEinzelbild umfassenden Zwischenbild gebildet, wobei das Einzelbild vor dem Hinzufügen in eine Bildebene des Zwischenbilds projiziert wird oder das Zwischenbild vor dem Hinzufügen in die Hauptbildebene des Einzelbilds projiziert wird.

Alternativ wird eine Gesamtbildebene festgelegt und alle Einzelbilder vor dem Hinzufügen in die Gesamtbildebene projiziert. Entsprechend liegt die Bildebene des Zwischenbilds in der Gesamtbildebene bzw. fällt mit dieser zusammen.

Vorteilhafterweise wird für jedes Einzelbild ein Fixpunkt im Raum bestimmt, wobei ein zumindest die meisten Fixpunkte umfassender Pfad im Raum berechnet wird, zu auf dem Pfad liegenden Fixpunkten gehörende Einzelbilder ausgewählt werden und das Gesamtbild aus den ausgewählten Einzelbildern errechnet wird.

Mittels des Pfads wird eine Auswahl an Einzelbildern getroffen, die zu dem Gesamtbild beitragen sollen. Einzelbilder, die beispielsweise aus zu weit von den restlichen Aufnahmen abweichenden Kamerapositionen aufgenommen wurden und/oder nach bzw. bei einer Rückwärtsbewegung der Kamera aufgenommen wurden, werden verworfen.

Vorteilhafterweise entspricht der Fixpunkt für ein Einzelbild einer Position der Kamera bei der Aufnahme des Einzelbilds oder einem auf einen Sensormittelpunkt einer aufnehmenden Kamera abgebildeten Bildpunkt des Einzelbilds in der Hauptbildebene oder einem Bildmittelpunkt des Einzelbilds in der Hauptbildebene.

Vorteilhafterweise wird der Pfad anhand der Abstände zwischen jeweils zwei Fixpunkten und anhand eines Richtungsvektors zwischen den zu den Fixpunkten gehörenden Einzelbildern ermittelt.

Alternativ wird eine globale Registrierkarte erstellt, d.h. es wird versucht jedes Einzelbild zu jedem anderen Einzelbild auszurichten und bei Erfolg die relative räumliche Ausrichtung und/oder ein Abstand zwischen den Einzelbildern in der Registrierkarte vermerkt. Anschließendwird anhand der vermerkten Ausrichtungen bzw. Abstände ein kürzester Pfad von einem ersten Einzelbildzueinemletzten Einzelbildgesucht und Bilder au ßerhalb des Pfades verworfen. Alternativ wird anhand der globalen Registrierkarte ein Spannbaum ermittelt und anschließend einzelne Äste verworfen.

Vorteilhafterweise wird für jedes zur Bildung des Gesamtbilds zu verwendende Einzelbild ein Bildmittelpunkt in der Hauptbildebene des Einzelbilds bestimmt, wobei die Bildmittelpunkte der Einzelbilder durch eine parametrisierte Kurve genähert werden und jedes Einzelbild vor dem Zusammenfügen zu dem Gesamtbild entlang einer ersten und einer zweiten Schnittkante zugeschnitten wird, wobei die erste und die zweite Schnittkante jeweils innerhalb der Hauptbildebene und senkrecht zu der Kurve verläuft.

Das Zuschneiden der Einzelbilder ermöglicht es insbesondere, unnötige oder sogar unerwünschte Überlappungen der Einzelbilder bei dem Zusammenfügen zu dem Gesamtbild zu vermeiden. Zwar sind Überlappungen für das Feststellen der relativen räumlichen Ausrichtung zwischen zwei Einzelbildern notwendig, bei einem Zusammenfügen können Überlappungen jedoch die Qualität des Gesamtbilds beeinträchtigen und werden durch ein geeignetes Zuschneiden der Einzelbilder vermieden.

Vorteilhafterweise entspricht der Bildmittelpunkt einem auf einen Sensormittelpunkt einer aufnehmenden Kamera abgebildeten Bildpunkt des Einzelbilds in der Hauptbildebene oder einem geometrischen Schwerpunkt des Einzelbilds in der Hauptbildebene .

Vorteilhafterweise weisen die erste Schnittkante und die zweite Schnittkante jeweils einen Abstand zu dem Bildmittelpunkt des Einzelbilds auf, wobei der Abstand zwischen 40% und 60% eines Abstands des Bildmittelpunkts zu dem auf der Kurve vorausgehenden bzw. nachfolgenden Bildmittelpunkt beträgt.

Gemäß einer ersten alternativen Ausführungsform werden die Schnittkanten so gewählt, dass benachbarte Einzelbilder genau entlang der Schnittkanten aneinander stoßen bzw. die Schnittkanten von benachbarten Einzelbildern zusammenfallen. In einer zweiten alternativen Ausführungsform sind die Schnittkanten so gewählt, dass sich die Einzelbilder in einem an die Schnittkante angrenzenden Bereich überlappen.

Vorteilhafterweise wird vor dem Zuschneiden der Einzelbilder mindestens ein Bildmittelpunkt entlang der Kurve oder parallel zu der Kurve innerhalb der Bildebene auf eine neue Position verschoben. Durch das Verschieben eines Bildmittelpunkts verschieben sich auch die relativ zu dem Bildmittelpunkt ermittelten Schnittkanten entsprechend. Das Verschieben ermöglicht **es,** Unterschiede hinsichtlich der Abstände zwischen benachbarten Bildmittelpunkten entlang der Kurve auszugleichen und die Qualität des Gesamtbilds zu verbessern.

Vorteilhafterweise umfasst jeder in einem der Einzelbilder erfasste erste Teilbereich mit mindestens einem weiteren in einem der weiteren Einzelbilder erfassten Teilbereich eine Überlappung von mindestens 30% oder mindestens 40% oder mindestens 50% des ersten Teilbereichs. Je größer die Überlappung, also der Anteil der Überlappung an dem jeweils erfassten Teilbereich, umso zuverlässiger und genauer lässt sich die relative räumliche Ausrichtung bestimmen.

Vorteilhafterweise wird ein sich an die Schnittkante angrenzender Teilbereich jedes Einzelbilds mit einem Teilbereich eines angrenzenden Einzelbilds überblendet. Bevorzugt werden die Teilbereiche in einer senkrecht zu der Schnittkante verlaufenden Richtung linear überblendet.

Vorteilhafterweise werden die Einzelbilder mittels einer 2D-intraoral-Kamera oder einer 3D-intraoral-Kamera erfasst.

### Kurzbeschreibung der Zeichnungen

Ausführungsbeispiele der Erfindung sind in den Zeichnungen dargestellt. Es zeigt die
- Fig. 1: einen Ablauf gemäß einer ersten Ausführungsform,
- Fig. 2: einen gemäß einer erfindungsgemäßen Ausführung ermittelten Pfad,
- Fig. 3: ein Ermitteln von Schnittkanten für Einzelbilder gemäß einer Weiterbildung,
- Fig. 4A,B: eine Skizze zur Verdeutlichung der erfindungsgemäßen Projektion.

### Ausführungsbeispiele

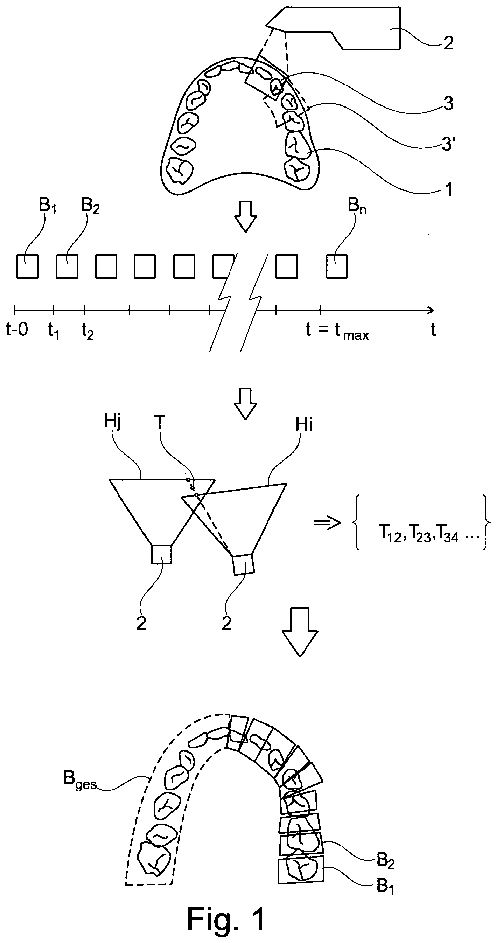

In Fig. 1 sind Verfahrensschritte gemäß einer ersten Ausführungsform des erfindungsgemäßen Verfahrens schematisch dargestellt.

Im Ausführungsbeispiel wird ein Unterkiefer als Aufnahmebereich 1 mittels einer intraoralen Kamera 2 erfasst. Hierfür wird die Kamera 2 während eines Aufnahmezeitintervalls t=0 bis t=tmax über den Unterkiefer 1 bewegt, wobei nacheinander Teilbereiche 3 des Aufnahmebereichs 1 jeweils in Einzelbildern Bi, i= 1...N erfasst werden.

Jedes Einzelbild Bi umfasst eine Matrix aus n x m Bildpunkten, wobei jeder Bildpunkt einen Grauwert umfasst. Gemäß alternativer Ausführungsformen weist jeder Bildpunkt einen Farbwert oder mehrere Farbwerte oder einen Höhenwert auf.

Um anhand der mehreren Einzelbilder Bi ein zweidimensionales Gesamtbild Bges des Aufnahmebereichs 1 zu erzeugen, wird für jedes Einzelbild Bi eine relative räumliche Ausrichtung zu mindestens einem weiteren Einzelbild Bj ermittelt, wobei das Einzelbild Bi und das weitere Einzelbild Bj nicht identisch sind, jedoch der in dem Einzelbild Bierfasste Teilbereich 3 mit einem in demweiteren Einzelbild Bj erfassten Teilbereich 3' zumindest überlappt. Die beiden Einzelbilder Bi und Bj sind beispielsweise zwei zeitlich aufeinanderfolgend aufgenommene Bilder.

Die relative Ausrichtung der beiden Einzelbilder Bi und Bj zueinander wird mittels eines Optimierungsverfahrens ermittelt. Hierfür wird für beide Einzelbilder Bi und Bj jeweils von einer schärfsten Ebene als Hauptbildebene Hi bzw. Hj und von einer ersten relativen räumlichen Ausrichtung der beiden Hauptbildebenen Hi und Hj zueinander ausgegangen.

Gemäß dieser ersten räumlichen Ausrichtung wird das erste Einzelbild Bi auf das weitere Einzelbild Bj projiziert. Die Projektion kann genauso umgekehrt vorgenommen werden. Die Projektion erfolgt durch eine Transformation Tij der Bildpunkte des ersten Einzelbilds Bi aus der Hauptbildebene Hi in die Hauptbildebene Hj des weiteren Einzelbilds Bj, wodurch zumindest zu einem Teil der Bildpunkte des ersten Einzelbilds Bi jeweils entsprechende Bildpunkte im weiteren Einzelbild Bj ermittelt werden. Für jedes Paar von sich entsprechenden Bildpunkten in dem ersten und dem weiteren Einzelbild Bi und Bj wird jeweils eine Differenz der zugehörigen Grauwerte berechnet und anhand der Differenz ein Qualitätswert gebildet.

Da solche Bildpunkte in dem Einzelbild Bj und dem weiteren Einzelbild Bi, die denselben Punkt des aufzunehmenden Bereichs oder Objekts erfassen, hinsichtlich des erfassten Werts übereinstimmen oder zumindest nur geringfügig voneinander abweichen, verringert sich die berechnete Differenz, je besser die angenommene Ausrichtung zwischen den Hauptbildebenen Hi und Hj der beiden Einzelbilder Bi und Bj mit der realen Ausrichtung der Einzelbilder Bi und Bj übereinstimmt. Somit kann durch ein Minimieren der berechneten Differenz bzw. ein entsprechendes Optimieren des Qualitätswerts die angenommene Ausrichtung an die reale Ausrichtung angenähert werden.

Der Qualitätswert wird entsprechend durch Veränderung der relativen räumlichen Ausrichtung des ersten und des weiteren Einzelbilds Bi und Bj zueinander optimiert bzw. einem vorgegebenen Zielwertangenähert. DerZielwert selbst kann als Abbruchbedingung für das Optimierungsverfahren dienen.

Ausgangspunkt des Optimierungsverfahrens ist eine erste relativen räumlichen Ausrichtung zwischen dem ersten und dem weiteren Einzelbild Bi und Bj, z.B. eine Identität der beiden Hauptbildebenen Hi und Hj, von der aus die relative räumliche Ausrichtung schrittweise durch ein relatives Verschieben und/oder ein relatives Drehen der einen Hauptbildebene Hi oder Hj gegenüber der anderen Hauptbildebene Hj oder Hi verändert wird. Für jede veränderte räumliche Ausrichtung wirdwiederum das eine Einzelbild Bi oder Bj auf das andere Einzelbild Bj oder Bi projiziert und für Bildpunktpaare aus sich entsprechenden Bildpunkten der beiden Einzelbilder Bi und Bj wird die Differenz der Grauwerte berechnet und der Qualitätswert gebildet.

Mittels des Optimierungsverfahrens wird zu jedem Einzelbild Bi mindestens eine relative räumliche Ausrichtung zu einem weiteren Einzelbild Bj oder zu einem das weitere Einzelbild Bj umfassenden Zwischenbild ermittelt.

Das Gesamtbild Bges wird anhand der ermittelten relativen räumlichen Ausrichtungen zusammengefügt. Beispielsweise wird ein erstes Einzelbild B1 in die Hauptbildebene eines zweiten Einzelbilds B2 projiziert, die beiden Einzelbilder B1 und B2 zu einem ersten Zwischenbild zusammengefügt, anschließend das erste Zwischenbild in die Hauptbildebene eines dritten Einzelbilds B3 projiziert, das erste Zwischenbild und das dritte Einzelbild zu einem zweiten Zwischenbild zusammengefügt usw.

Als erste Einzelbild B1 wird beispielsweise das zuerst oder zuletzt erfasste Einzelbild oder ein zu einem anderen optimalen Zeitpunkt aufgenommenes Einzelbild ausgewählt. Oder es wird ein einen ersten oder zweiten Rand des Aufnahmebereichs 1 erfassendes Einzelbild oder ein einen mittleren Teilbereich 3 des Aufnahmebereichs 1 erfassendes Einzelbild als erstes Einzelbild ausgewählt. Durch dieauswahl eines Einzelbilds, welches einenmittleren Teilbereich 3 des Aufnahmebereichs 1 erfasst können Verzerrungen verringert oder vermieden werden.

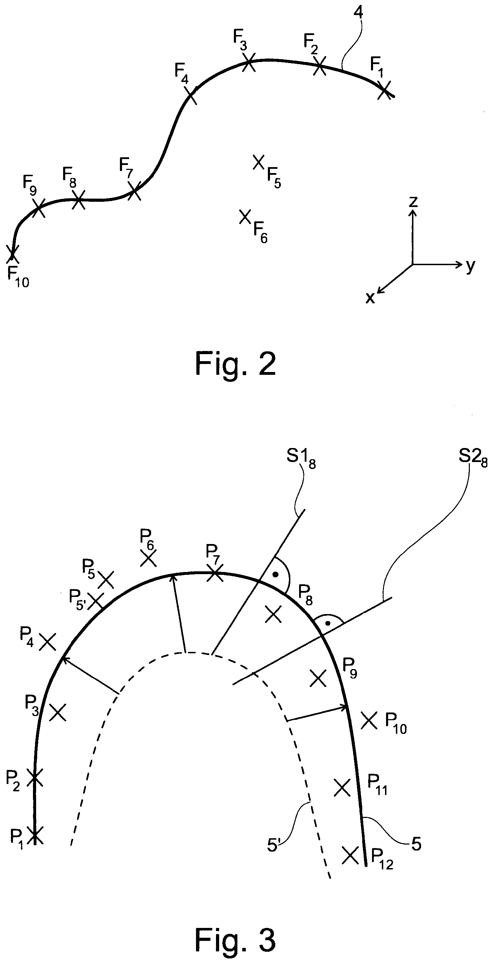

Gemäß einer Weiterbildung, werden nicht alle Einzelbilder Bi für das Gesamtbild Bges verwendet, sondern es wird eine Auswahl getroffen bzw. ein Teil der Einzelbilder Bi wird verworfen. Hierfür wird, wie in Fig. 2 schematisch dargestellt, ein Pfad 4 berechnet. Ausgangspunkt für den Pfad bilden Fixpunkte Fi, wobei zu jedem Einzelbild Bi ein Fixpunkt Fi berechnet wird. Gemäß einer ersten Ausführungsform repräsentieren die Fixpunkte Fi zu jedem Einzelbild Bi jeweils eine dreidimensionale Kameraposition, aus der das Einzelbild Bi erfasst wurde. Die Kameraposition wird jeweils anhand des Einzelbilds Bi und der bestimmten relativen räumlichen Ausrichtung der dazugehörigen Hauptbildebene Hi bestimmt.

Anschließend wird der Pfad 4 als eine Verbindungslinie von einem ersten Fixpunkt F1 zu einem letzten Fixpunkt FN ermittelt, wobei Ausreißer oder Rückwärtsbewegungen hinsichtlich der weiteren Fixpunkte Fi, i=2...N-1, verworfen werden.

Der Pfad 4 wird beispielsweise anhand von Kostenfunktionen ermittelt, wobei für jede Verbindung von einem ersten Fixpunkt Fi zu einem weiteren Fixpunkt Fj eine Kostenfunktion aufgestellt wird. DieKostenfunktion berücksichtigt beispielsweise sowohl den Abstand zwischen dem ersten Fixpunkt Fi und dem weiteren Fixpunkt Fj als auch einen Richtungsvektor zwischen der Hauptbildebene Hi des zu dem ersten Fixpunkt Fi gehörenden ersten Einzelbilds Bi und der Hauptbildebene Hj des zu dem weiteren Fixpunkt Fj gehörenden weiteren Einzelbilds Bj. Als Richtungsvektor dient beispielsweise der translatorische Anteil einer Projektion der Hauptbildebene Hi des einen Einzelbilds Bi auf die Hauptbildebene Hj des weiteren Einzelbilds Hj.

Der anhand der Kostenfunktionen bestimmte Pfad 4 durchläuft typischerweise die meisten Fixpunkte Fi zwischen dem ersten Fixpunkt F1 und dem letzten Fixpunkt FN, wobei stark abweichende oder rückläufige Fixpunkte Fi nicht von dem Pfad 4 durchlaufen werden.

Das Gesamtbild wird anschließend nur aus solchen Einzelbildern Bi zusammengesetzt, deren Fixpunkte Fi von dem Pfad 4 durchlaufen werden. Die Einzelbilder Bi, deren Fixpunkte Fi nicht von dem Pfad 4 durchlaufen werden, werden verworfen.

Gemäß einer weiteren Ausführungsform werden die für das Gesamtbild Bges verwendeten Einzelbilder Bi vor dem Zusammenfügen zugeschnitten, d.h. es wird jeweils nur ein Teil bzw. Ausschnitt jedes Einzelbilds Bi für das Gesamtbild Bges verwendet. Hierfür werden für jedes Einzelbild Bi zwei Schnittkanten S1i und S2i ermittelt, wie es schematisch in Fig. 3 dargestellt ist. Für alle für das Gesamtbild Bges zu verwendenden Einzelbilder Bi wird ein Referenzbildpunkt Pi bestimmt, z.B. ein jeweiliger Bildmittelpunkt. Die Referenzbildpunkte Pi werden anhand der bestimmten relativen räumlichen Ausrichtung der jeweiligen Hauptbildebenen Hi in ein gemeinsames Koordinatensystem transformiert und in dem gemeinsamen Koordinatensystem durch eine Kurve 5 mit einer vorbestimmten Grundform, z.B. eine Parabel oder eine Hyperbel oder einen Kreis, angenähert. Wie in Fig. 3 skizziert wird beispielsweise mit einer Kurve 5' mit einem ersten Parametersatz gestartet. Anschließend werden die Abstände zu den Referenzbildpunkten Pi durch Änderung der Parameter des Parametersatzes minimiert. Das Ergebnis des Minimierens ist die Kurve 5.

Die Schnittkanten S1i und S21 werden anhand der Kurve 5 als senkrecht zu der Kurve 5 verlaufende Linien ermittelt. Handelt es sich bei den Referenzbildpunkten Pi jeweils um den Mittelpunkt des jeweiligen Einzelbilds Bi, so wird die Schnittkante S1i bzw. S2i beispielsweise so gewählt, dass sie durch einen jeweils in gleichem Abstand zu den Referenzbildpunkten Pi von zwei benachbarten Einzelbildern Bi und Bi+1 auf der Kurve liegenden Punkt verläuft.

Gemäß einer Weiterbildung werden vor der Ermittlung der Schnittkanten S1i und S2i die Positionen der Referenzbildpunkte Pi auf der Kurve 5 bzw. die Abstände der Referenzbildpunkte Pi zueinander geprüft und einzelne Referenzbildpunkte Pi gegebenenfalls verschoben, um möglichst gleichmäßige Abstände zwischen allen aufeinanderfolgenden Referenzbildpunkten Pi entlang der Kurve zu erhalten. In Fig. 3 ist dies beispielhaft für den Referenzbildpunkt P5 dargestellt, der zu Referenzbildpunkt P5' verschoben wird. Der Abstand zwischen zu dicht beieinanderliegenden Referenzbildpunkte Pi wird durch Verschieben von mindestens einem der beiden Referenzbildpunkte Pi entlang der Kurve 5 künstlich vergrößert. Die Schnittkanten S1i und S2i werden anschließend unter Berücksichtigung der neuen Position des Referenzbildpunkts Pi berechnet.

Die Schnittkanten S1i und S2i werden so gewählt, dass Schnittkanten von jeweils benachbarten Einzelbildern Bi und Bi+1 zusammenfallen bzw. aneinander angrenzen. Alternativ werden die Schnittkanten S1i und S2i so gewählt, dass jeweils benachbarte Einzelbilder Bi und Bi+1 eine Überlappung einer vorgegebenen Breite aufweisen, wobei es vorteilhaft ist, die beiden Einzelbilder Bi und Bi+1 bei dem Zusammenfügen im Bereich der Überlappung insbesondere linear zu überblenden, d.h. die Werte der Bildpunkte der Einzelbilder werden im Bereich der Überlappung in Richtung senkrecht zu der jeweiligen Schnittkante um einen zunehmenden Faktor reduziert, bevor die Einzelbilder zu dem Gesamtbild zusammengefügt werden.

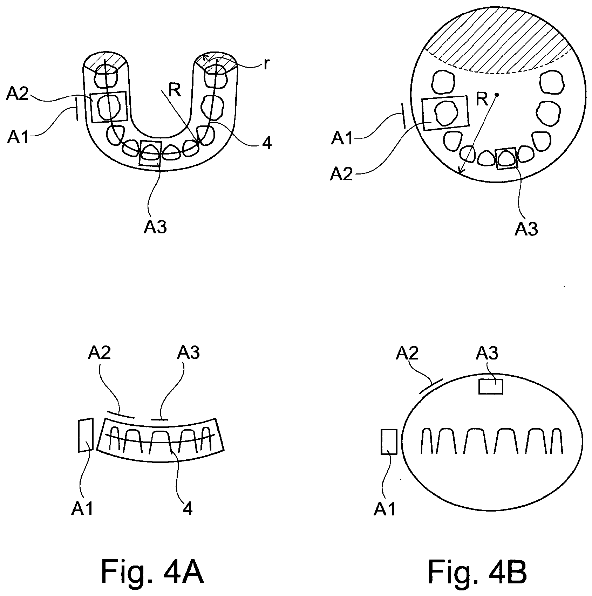

Anhand der Skizze gemäß Fig. 4A und 4B wird der Unterschied zwischen der Projektion auf einen Torus im 3-dimensionalen Raum und der Projektion auf eine Kugel erläutert. Ein Torus im 3-dimensionalen Raum ist die Menge der Punkte, die von einer Kreislinie mit Radius R den festen Abstand r mit r < R haben. Eine Kugel im 3-dimensionalen Raum ist die Menge der Punkte, die von einem Mittelpunkt den festen Abstand R haben.

In Fig. 4A ist der Torus und in Fig. 4B die Kugel zu sehen, im oberen Teil in der Ansicht von oben und im unteren Teil in der Ansicht von vorne. Genau genommen handelt es sich um eine Hälfte eines Torus, die durch einen Schnittebene senkrecht zur Kreislinie mit Radius R entstanden ist und um eine Halbkugel.

Es sind jeweils drei Aufnahmen A1, A2 und A3 skizziert. Bei der Aufnahme A2 und A3 ist zu sehen, dass es Unterschiede bei der Projektion zwischen Kugel und Torus gibt, wobei bei der Kugel die Aufnahmen stärker verzerrt werden als bei dem Torus.

Die Kreislinie mit Radius R des Torus durch den Pfad 4 der Aufnahme bestimmt, sodass der Pfad 4 gleichzeitig das Skelett des Torus ist. Der Abstand r des Torus mit r< R kann ebenfalls aus denAufnahmen ermittelt werden oder vorgegeben sein. Somit ist erst am Ende der Aufnahme Torus vollständig definiert.

### Bezugszeichenliste

- 1: Aufnahmebereich
- 2: Kamera
- 3: Teilbereich des Aufnahmebereichs
- 4: Pfad
- 5: Kurve
- 5': Kurve
- A1: Aufnahme 1
- A2: Aufnahme 2
- A3: Aufnahme 3
- Bi: Einzelbild
- Bges: zweidimensionales Gesamtbild
- Fi: Fixpunkt
- Hi: Hauptbildebene
- Pi: Referenzbildpunkt
- R: Radius der Kreislinie des Torus bzw. der Kugel
- r: Abstand zur Kreislinie
- T: Transformation

## Patentansprüche

1. Computer-implementiertes Verfahren zum Erfassen mehrerer Einzelbilder (Bi) eines Aufnahmebereichs (1) eines Objekts mittels einer 2D oder 3D intraoralen Kamera (2) und zur Erzeugung eines zweidimensionalen Gesamtbilds (Bges) des mittels mehrerer Einzelbilder (Bi) erfassten Aufnahmebereichs (1), wobei
- jedes Einzelbild (Bi;Bj) einen Teilbereich (3;3') des Aufnahmebereichs (1) aus einer eigenen Blickrichtung und mit einem eigenen Abstand zu dem Teilbereich (3;3') erfasst,
- jedes Einzelbild (Bi;Bj) eine Vielzahl von Bildpunkten mit mindestens einem Farbwert und/oder mindestens einem Grauwert umfasst,
- der in jedem Einzelbild (Bi;Bj) jeweils erfasste Teilbereich (3;3') mit mindestens einem Teilbereich (3;3') in jeweils mindestens einem weiteren Einzelbild (Bi;Bj) überlappt,
wobei
- eine räumliche Ausrichtung einer Hauptbildebene (Hi;Hj) jedes Einzelbilds (Bi;Bj) zu einer Hauptbildebene (Hi;Hj) des jeweiligen weiteren Einzelbilds (Bi;Bj) anhand der Überlappung der jeweils erfassten Teilbereiche (3; 3') ermittelt wird und
- zumindest eine Vielzahl der Einzelbilder (Bi;Bj) entsprechend der ermittelten jeweiligen räumlichen Ausrichtungen zueinander zu dem Gesamtbild (Bges) zusammengesetzt werden,
**dadurch gekennzeichnet, dass**
- die räumliche Ausrichtung der Hauptbildebene (Hi;Hj) jedes Einzelbilds (Bi;Bj) zu der Hauptbildebene (Hi; Hj) des weiteren Einzelbilds (Hi;Hj) durch Optimieren eines Qualitätswerts ermittelt wird, wobei
- für eine erste relative räumliche Ausrichtung zu mindestens einem ersten Bildpunkt in der Hauptbildebene (Hi;Hj) des Einzelbilds (Bi;Bj) ein gemäß der Ausrichtung entsprechender Bildpunkt in der Hauptbildebene (Hi;Hj) des weiteren Einzelbilds (Bi;Bj) ermittelt wird,
- ein Vergleichswert der jeweiligen Farbwerte und/oder Grauwerte des Bildpunkts des Einzelbilds (Bi;Bj) und des entsprechenden Bildpunkts des weiteren Einzelbilds (Bi;Bj) gebildet wird,
- aus dem mindestens einen Vergleichswert der Qualitätswert gebildet wird und
- der Qualitätswert durch Verändern der relativen räumlichen Ausrichtung der Hauptbildebene (Hi;Hj) des Einzelbilds (Bi;Bj) zu der Hauptbildebene (Hi;Hj) des weiteren Einzelbilds (Bi;Bj) einem vorgegebenen Zielwert zumindest angenähert wird, wobei
- mindestens ein Bildpunktepaar aus Bildpunkt des Einzelbilds (Bi;Bj) und entsprechendem Bildpunkt des weiteren Einzelbilds (Bi;Bj) durch eine Projektion des Einzelbilds (Bi;Bj) und des weiteren Einzelbilds (Bi;Bj) oder eines das weitere Einzelbild (Bi;Bj) umfassenden Zwischenbilds auf eine vorab festgelegte Gesamtbildfläche ermittelt wird, wobei die Projektion entsprechend der relativen räumlichen Ausrichtung sowie einer relativen räumlichen Ausrichtung des Einzelbilds (Bi;Bj) oder des weiteren Einzelbilds (Bi;Bj) zu der Gesamtbildfläche vorgenommen wird und wobei
- die Gesamtbildfläche die Oberfläche eines Torus ist.

2. Verfahren nach Anspruch 1, **dadurch gekennzeichnet, dass** die Einzelbilder (Bi;Bj) jeweils ein Farbbild der Kamera (1) oder Grauwertbild der Kamera (1) oder dreidimensionales Bild mit einer Textur sind.

3. Verfahren nach einem der Ansprüche 1 bis 2, **dadurch gekennzeichnet, dass** das Gesamtbild schrittweise durch Hinzufügen des Einzelbilds zu einem zumindest das weitere Einzelbild umfassenden Zwischenbild gebildet wird, wobei das Einzelbild vor dem Hinzufügen in eine Bildebene des Zwischenbilds projiziert wird oder das Zwischenbild vor dem Hinzufügen in die Hauptbildebene des Einzelbilds projiziert wird.

4. Verfahren nach einem der Ansprüche 1 bis 2, **dadurch gekennzeichnet, dass** das Gesamtbild schrittweise durch Hinzufügen des Einzelbilds zu einem zumindest das weitere Einzelbild umfassenden Zwischenbild gebildet wird, wobei eine Gesamtbildfläche festgelegt wird und alle Einzelbilder vor dem Hinzufügen auf die Gesamtbildfläche projiziert werden.

5. Verfahren nach einem der Ansprüche 1 bis 4, **dadurch gekennzeichnet, dass**
- für jedes Einzelbild ein Fixpunkt (Fi) im Raum bestimmt wird,
- ein zumindest die meisten Fixpunkte (Fi) umfassender Pfad (4) im Raum berechnet wird,
- zu auf dem Pfad (4) liegenden Fixpunkten (Fi) gehörende Einzelbilder ausgewählt werden und
- das Gesamtbild aus den ausgewählten Einzelbildern errechnet wird.

6. Verfahren nach Anspruch 5, **dadurch gekennzeichnet, dass** der Fixpunkt (Fi) für ein Einzelbild einer Position der Kamera (1) bei der Aufnahme des Einzelbilds oder einem auf einen Sensormittelpunkt der aufnehmenden Kamera (1) abgebildeten Bildpunkt des Einzelbilds in der Hauptbildebene oder einem Bildmittelpunkt des Einzelbilds in der Hauptbildebene entspricht.

7. Verfahren nach Anspruch 5 oder 6, **dadurch gekennzeichnet, dass** der Pfad (4) anhand der Abstände zwischen jeweils zwei Fixpunkten und anhand eines Richtungsvektors zwischen den zu den Fixpunkten gehörenden Einzelbildern ermittelt wird.

8. Verfahren nach einem der Ansprüche 1 bis 7, **dadurch gekennzeichnet, dass** für jedes zur Bildung des Gesamtbilds zu verwendende Einzelbild ein Bildmittelpunkt in der Hauptbildebene des Einzelbilds bestimmt wird, die Bildmittelpunkte der Einzelbilder durch eine parametrisierte Kurve genähert werden und jedes Einzelbild vor dem Zusammenfügen zu dem Gesamtbild entlang einer ersten und einer zweiten Schnittkante (S1i; S2i) zugeschnitten wird, wobei die erste und die zweite Schnittkante jeweils innerhalb der Hauptbildebene und senkrecht zu der Kurve (5) verläuft.

9. Verfahren nach Anspruch 8, **dadurch gekennzeichnet, dass** der Bildmittelpunkt einem auf einen Sensormittelpunkt der aufnehmenden Kamera (1) abgebildeten Bildpunkt des Einzelbilds in der Hauptbildebene oder einem geometrischen Schwerpunkt des Einzelbilds in der Hauptbildebene entspricht.

10. Verfahren nach einem der Ansprüche 8 oder 9, **dadurch gekennzeichnet, dass** die erste Schnittkante und die zweite Schnittkante jeweils einen Abstand zu dem Bildmittelpunkt des Einzelbilds aufweisen, wobei der Abstand zwischen 40% und 60% eines Abstands des Bildmittelpunkts zu dem auf der Kurve vorausgehenden bzw. nachfolgenden Bildmittelpunkt beträgt.

11. Verfahren nach einem der Ansprüche 8 bis 10, **dadurch gekennzeichnet, dass** vor dem Zuschneiden der Einzelbilder mindestens ein Bildmittelpunkt entlang der Kurve oder parallel zu der Kurve innerhalb der Bildebene auf eine neue Position verschoben wird.

12. Verfahren nach einem der Ansprüche 1 bis 11, **dadurch gekennzeichnet, dass** jeder in einem der Einzelbilder erfasste erste Teilbereich mit mindestens einem weiteren in einem der weiteren Einzelbilder erfassten Teilbereich einen Überlapp von mindestens 30% oder mindestens 40% oder mindestens 50% des ersten Teilbereichs umfasst.

13. Verfahren nach einem der Ansprüche 1 bis 12, **dadurch gekennzeichnet, dass** ein sich an die Schnittkante angrenzender Teilbereich jedes Einzelbilds mit einem Teilbereich eines angrenzenden Einzelbilds überblendet wird.

14. Verfahren nach Anspruch 13, **dadurch gekennzeichnet, dass** die Teilbereiche in einer senkrecht zu der Schnittkante verlaufenden Richtung linear überblendet werden.

## Claims

1. Computer-implemented method for capturing a plurality of individual images (Bi) of a capture area (1) of an object by means of a 2D or 3D intraoral camera (2) and for producing a two-dimensional whole image (Bges) of the capture area (1) captured by means of a plurality of individual images (Bi), wherein
- each individual image (Bi;Bj) captures a partial area (3;3') of the capture area (1) from its own viewing direction and at its own distance from the partial area (3;3'),
- each individual image (Bi;Bj) comprises a plurality of pixels with at least one color value and/or at least one gray value,
- the partial area (3;3') captured in each individual image (Bi;Bj) overlaps with at least one partial area (3;3') in at least one further individual image (Bi;Bj), wherein
- a spatial orientation of a main image plane (Hi;Hj) of each individual image (Bi;Bj) to a main image plane (Hi;Hj) of the respective further individual image (Bi;Bj) is determined on the basis of the overlap of the respective captured partial areas (3;3') and
- at least a plurality of the individual images (Bi;Bj) are combined to form the whole image (Bges) in accordance with the determined respective spatial orientations with respect to one another,
**characterized in that**
- the spatial orientation of the main image plane (Hi;Hj) of each individual image (Bi;Bj) to the main image plane (Hi; Hj ) of the further individual image (Hi;Hj) is determined by optimizing a quality value, wherein
- for a first relative spatial orientation to at least one first pixel in the main image plane (Hi;Hj) of the individual image (Bi;Bj), a pixel corresponding to the orientation is determined in the main image plane (Hi;Hj) of the further individual image (Bi;Bj),
- a comparison value of the respective color values and/or gray values of the pixel of the individual image (Bi;Bj) and of the corresponding pixel of the further individual image (Bi;Bj) is formed,
- the quality value is formed from the at least one comparison value, and
- the quality value is at least approximated to a predetermined target value by changing the relative spatial orientation of the main image plane (Hi;Hj) of the individual image (Bi; Bj) to the main image plane (Hi; Hj) of the further individual image (Bi;Bj), wherein
- at least one pixel pair comprising a pixel of the individual image (Bi;Bj) and a corresponding pixel of the further individual image (Bi;Bj) is determined by a projection of the individual image (Bi;Bj) and of the further individual image (Bi;Bj) or of an intermediate image comprising the further individual image (Bi;Bj), onto a predetermined total whole image area, wherein the projection is carried out in accordance with the relative spatial orientation and a relative spatial orientation of the individual image (Bi;Bj) or of the further individual image (Bi;Bj) to the whole image area and wherein
- the whole image area is the surface of a torus.

2. Method according to claim 1, **characterized in that** the individual images (Bi;Bj) are each a color image of the camera (1) or a grayscale image of the camera (1) or a three-dimensional image with a texture.

3. Method according to one of claims 1 to 2, **characterized in that** the whole image is formed gradually by adding the individual image to an intermediate image comprising at least the further individual image, wherein the individual image is projected into an image plane of the intermediate image before being added, or the intermediate image is projected into the main image plane of the individual image before being added.

4. Method according to one of claims 1 to 2, **characterized in that** the whole image is formed gradually by adding the individual image to an intermediate image comprising at least the further individual image, wherein a whole image area is defined and all individual images are projected onto the whole image area before being added.

5. Method according to one of claims 1 to 4, **characterized in that**
- for each individual image, a fixed point (Fi) is determined in space,
- a path (4) comprising at least most of the fixed points (Fi) is calculated in space,
- individual images belonging to fixed points (Fi) lying on the path (4) are selected and
- the whole image is calculated from the individual images selected.

6. Method according to claim 5, **characterized in that** the fixed point (Fi) for an individual image corresponds to a position of the camera (1) during the capture of the individual image or a pixel of the individual image imaged on a sensor center point of the capturing camera (1) in the main image plane or an image center point of the individual image in the main image plane.

7. Method according to claim 5 or 6, **characterized in that** the path (4) is determined on the basis of the distances between two fixed points and on the basis of a direction vector between the individual images belonging to the fixed points.

8. Method according to one of claims 1 to 7, **characterized in that** for each individual image to be used to form the whole image, an image center point in the main image plane of the individual image is determined, the image center points of the individual images are approximated by a parameterized curve, and each individual image is cropped along a first and a second cutting edge (S1i;S2i) before being joined together to form the whole image, wherein the first and the second cutting edge each run within the main image plane and perpendicular to the curve (5).

9. Method according to claim 8, **characterized in that** the image center point corresponds to a pixel of the individual image imaged on a sensor center point of the capturing camera (1) in the main image plane or a geometric main focus of the individual image in the main image plane.

10. Method according to one of claims 8 or 9, **characterized in that** the first cutting edge and the second cutting edge each have a distance from the image center point of the individual image, wherein the distance is between 40% and 60% of a distance of the image center point from the image center point preceding or following on the curve.

11. Method according to one of the claims 8 to 10, **characterized in that** at least one image center point is shifted to a new position along the curve or parallel to the curve within the image plane before the individual images are cropped.

12. Method according to one of claims 1 to 11 **characterized in that** each first partial area captured in one of the individual images comprises an overlap of at least 30% or at least 40% or at least 50% of the first partial area having at least one further partial area captured in one of the further individual images.

13. Method according to one of claims 1 to 12, **characterized in that** a partial area of each individual image adjacent to the cutting edge is faded over with a partial area of an adjacent individual image.

14. Method according to Claim 13, **characterized in that** the partial areas are faded over linearly in a direction running perpendicular to the cutting edge.

## Revendications

1. Procédé mis en œuvre par ordinateur permettant l'acquisition de plusieurs images individuelles (Bi) d'une zone d'enregistrement (1) d'un objet au moyen d'une caméra intra-buccale 2D ou 3D (2) et permettant la génération d'une image globale bidimensionnelle (Bges) de la zone d'enregistrement (1) acquise au moyen de plusieurs images individuelles (Bi),
- chaque image individuelle (Bi ; Bj) acquérant une zone partielle (3, 3') de la zone d'enregistrement (1) selon une direction de visée propre et à une distance propre par rapport à la zone partielle (3 ; 3'),
- chaque image individuelle (Bi ; Bj) comprenant une pluralité de pixels présentant au moins une valeur de couleur et/ou au moins une valeur de gris,
- la zone partielle (3 ; 3') acquise dans chaque image individuelle (Bi ; Bj) chevauchant au moins une zone partielle (3 ; 3') dans au moins une autre image individuelle (Bi ; Bj),
- une orientation spatiale d'un plan d'image principal (Hi ; Hj) de chaque image individuelle (Bi ; Bj) par rapport à un plan d'image principal (Hi ; Hj) de l'autre image individuelle respective (Bi ; Bj) étant déterminée sur la base du chevauchement des zones partielles (3 ; 3') respectivement acquises, et
- au moins une pluralité des images individuelles (Bi ; Bj) étant combinées de manière à former l'image globale (Bges) conformément aux orientations spatiales respectives déterminées les unes par rapport aux autres,
**caractérisé en ce que**
- l'orientation spatiale du plan d'image principal (Hi ; Hj) de chaque image individuelle (Bi ; Bj) par rapport au plan d'image principal (Hi ; Hj) de l'image individuelle (Hi ; Hj) est déterminée en optimisant une valeur de qualité,
- pour une première orientation spatiale relative, au moins un premier pixel dans le plan d'image principal (Hi ; Hj) de l'image individuelle (Bi ; Bj) étant déterminé par un pixel correspondant à l'orientation dans le plan d'image principal (Hi ; Hj) de l'autre image individuelle (Bi ; Bj),
- une valeur de comparaison des valeurs de couleur respectives et/ou des valeurs de gris du pixel de l'image individuelle (Bi ; Bj) et du pixel correspondant de l'autre image (Bi ; Bj) étant formée,
- la valeur de qualité étant formée à partir de ladite au moins une valeur de comparaison,
et
- la valeur de qualité étant au moins approximée à une valeur cible prédéfinie en modifiant l'orientation spatiale relative du plan d'image principal (Hi ; Hj) de l'image individuelle (Bi ; Bj) par rapport au plan d'image principal (Hi ; Hj) de l'autre image individuelle (Bi ; Bj),
- au moins une paire de pixels parmi le pixel de l'image individuelle (Bi ; Bj) et le pixel correspondant de l'autre image individuelle (Bi ; Bj) étant déterminée par une projection de l'image individuelle (Bi ; Bj) et de l'autre image individuelle (Bi ; Bj) ou d'une image intermédiaire comprenant l'autre image individuelle (Bi ; Bj) sur une surface d'image globale prédéfinie, ladite projection étant effectuée en fonction de l'orientation spatiale relative ainsi que d'une orientation spatiale relative de l'image individuelle (Bi ; Bj) ou de l'autre image individuelle (Bi ; Bj) par rapport à la surface d'image globale, et
- ladite surface d'image globale étant la surface d'un tore.

2. Procédé selon la revendication 1, **caractérisé en ce que** les images individuelles (Bi ; Bj) sont chacune une image couleur de la caméra (1) ou une image en niveaux de gris de la caméra (1) ou une image tridimensionnelle avec une texture.

3. Procédé selon l'une des revendications 1 à 2, **caractérisé en ce que** l'image globale est formée étape par étape en ajoutant l'image individuelle à une image intermédiaire comprenant au moins l'autre image individuelle, ladite image individuelle étant projetée dans un plan d'image de l'image intermédiaire avant l'ajout ou ladite image intermédiaire étant projetée dans le plan d'image principal de l'image individuelle avant l'ajout.

4. Procédé selon l'une des revendications 1 à 2, **caractérisé en ce que** l'image globale est formée étape par étape en ajoutant l'image individuelle à l'image intermédiaire comprenant au moins l'autre image individuelle, ladite surface d'image globale étant définie et toutes les images individuelles étant projetées sur la surface d'image globale avant l'ajout.

5. Procédé selon l'une des revendications 1 à 4, **caractérisé en ce que**
- pour chaque image individuelle, un point fixe (Fi) dans l'espace est déterminé,
- un trajet (4) comprenant au moins la plupart des points fixes (Fi) est calculé dans l'espace,
- des images individuelles appartenant à des points fixes (Fi) se trouvant sur le trajet (4) sont sélectionnées,
- l'image globale est calculée à partir des images individuelles sélectionnées.

6. Procédé selon la revendication 5, **caractérisé en ce que** le point fixe (Fi) pour une image individuelle correspond à la position de la caméra (1) lors de l'acquisition de l'image individuelle ou à un pixel de l'image individuelle imagé sur le centre de capteur de la caméra d'enregistrement (1) dans le plan d'image principal ou le centre d'image de l'image individuelle dans le plan d'image principal.

7. Procédé selon la revendication 5 ou 6, **caractérisé en ce que** le trajet (4) est déterminé sur la base de la distance entre deux points fixes respectifs et sur la base du vecteur directionnel entre les images individuelles appartenant aux points fixes.

8. Procédé selon l'une des revendications 1 à 7, **caractérisé en ce que**, pour chaque image individuelle devant être utilisée pour former l'image globale, le centre d'image est déterminé dans le plan d'image principal de l'image individuelle, les centres d'image des images individuelles sont approximés par une courbe paramétrée, et chaque image individuelle est déplacée le long d'un premier et d'un second bord de coupe (S1i ; S2i), lesdits premier et second bords de coupe s'étendant chacun au sein du plan d'image principal et perpendiculairement à la courbe (5).

9. Procédé selon la revendication 8, **caractérisé en ce que** le centre d'image correspond à un pixel de l'image individuelle représenté sur le centre de capteur de la caméra d'enregistrement (1) dans le plan d'image principal ou au centre de gravité géométrique de l'image individuelle dans le plan d'image principal.

10. Procédé selon l'une des revendications 8 ou 9, **caractérisé en ce que** le premier bord de coupe et le second bord de coupe présentent chacun une distance par rapport au centre d'image de l'image individuelle, ladite distance représentant entre 40 % et 60 % de la distance du centre d'image au centre d'image précédent ou suivant sur la courbe.

11. Procédé selon l'une des revendications 8 à 10, **caractérisé en ce qu'**avant la découpe sur mesure des images individuelles au moins un centre d'image est déplacé le long de la courbe ou parallèlement à la courbe au sein du plan d'image jusqu'à une nouvelle position.

12. Procédé selon l'un des revendications 1 à 11, **caractérisé en ce que** chaque première zone partielle acquise dans l'une des images individuelles comprend un chevauchement d'au moins 30 % ou d'au moins 40 % ou d'au moins 50 % de la première sous-zone, avec au moins une autre zone partielle acquise dans l'une des autres images individuelles.

13. Procédé selon l'une quelconque des revendications 1 à 12, **caractérisé en ce qu'**une zone partielle de chaque image individuelle adjacente au bord de coupe est superposée sur une zone partielle d'une image individuelle adjacente.

14. Procédé selon la revendication 13, **caractérisé en ce que** les zones partielles sont linéairement superposée dans une direction perpendiculaire au bord de coupe.
